# EUROPEAN PATENT SPECIFICATION

(11) **EP 1 976 571 B1**
(45) Date of publication and mention of the grant of the patent: **02.12.2009**
(21) Application number: 07710217.6
(22) Date of filing: 19.01.2007
(51) Int. Cl.: A61L 12/14, C11D 3/00

(54) **IMPROVING DISINFECTION EFFICACY OF LENS CARE REGIMEN FOR RIGID GAS PERMEABLE CONTACT LENSES**
VERBESSERUNG DER DESINFEKTIONSWIRKSAMKEIT VON LINSENPFLEGEPROGRAMMEN FÜR STARRE GASDURCHLÄSSIGE KONTAKTLINSEN
AMÉLIORATION DE L'EFFICACITÉ DE LA DÉSINFECTION DE VERRES DE CONTACT POUR DES VERRES DE CONTACT RIGIDES PERMÉABLES AUX GAZ

(30) Priority: 20.01.2006 US 760879 P; 20.01.2006 US 760436 P
(43) Date of publication of application: 08.10.2008
(73) Proprietor: Bausch & Lomb Incorporated, Rochester, NY 14604-2701 (US)
(72) Inventor: XIA, Erning, Penfield, NY 14526 (US); SALAMONE, Joseph, C., Boca Raton, FL 33486 (US); AMMON, Daniel, M., Penfield, NY 33486 (US); BORAZJANI, Roya, N., Ft. Worth, TX 76132 (US)
(74) Representative: Glas, Holger
(86) International application number: PCT/US2007/060751
(87) International publication number: WO 2007/084975

(56) References cited:
- WO-A-2004/112848
- US-A1- 2004 185 028
- US-A1- 2005 119 221
- US-A1- 2005 261 148

## Description

### Field

The present invention relates to a composition and method for disinfecting rigid gas permeable (RGP) contact lenses. Particularly, the present invention relates to a composition and method for disinfecting RGP contact lenses that require no lens rubbing step.

### Background

Contact lenses in use today fall into three general categories: hard lenses formed from materials prepared by polymerization of acrylic esters such as poly(methyl methacrylate) (PMMA); rigid gas permeable (RGP) lenses formed from silicone (meth)acrylates and fluorosilicone methacrylates; and gel, hydrogel or like soft-type lenses. The hard and rigid-type lenses, because they are characterized by low vapor diffusion and absorb only minor amounts of aqueous fluids, have a lower tendency to bind ingredients used in contact lens care solutions. On the other hand, soft-type lenses have a greater tendency to bind active ingredients used in contact lens care solutions. Therefore, developing solutions designed for the treatment of soft-type lenses, whether made from the more traditional copolymers of 2-hydroxyethyl methacrylate (HEMA) or from the newer siloxane-containing hydrogel materials is especially challenging.

In the normal course of wearing contact lenses, tear film and debris consisting of proteinaceous, oily, sebaceous, and related organic matter have a tendency to deposit and build-up on lens surfaces. Many factors influence deposit formation, including patient to patient variation, lens material, care regimen, and environment. In general, relatively high water content ionic lens materials absorb more protein than relatively low water content or nonionic lens materials. As part of the routine care regimen, contact lenses must be cleaned to remove tear film deposits and debris. If deposits are not properly removed, both the wettability and optical clarity of the lenses are substantially reduced and wearer discomfort may result.

Further, contact lenses must also be disinfected to kill harmful microorganisms that may be present or grow on the lenses. Some of the most popular products for disinfecting lenses are multi-purpose solutions that can be used to clean, disinfect, and wet contact lenses, followed by direct insertion or placement on the eye, without rinsing. Obviously, the ability to use a single solution for contact lens care is an advantage. Such a solution, however, must be particularly gentle to the eye, since at least some of the solution will be on the lens when inserted or placed on the eye and will thereby come into direct contact with eye tissues.

Since multipurpose solutions are designed for use as a wetting agent and are further designed to be used without rinsing, it is imperative that the solution be ophthalmically safe for eye contact. This limits, to some extent, the type and concentration of both cleaning agents and antimicrobial agents or biocides that can be employed in the solution. For example, as can be readily understood, biocides or cleaners in a shampoo product may not be suitable for ophthalmic use. It has been a challenge to develop a formula that is, on the one hand, maximally efficacious and, on the other hand, sufficiently gentle to be not only safe, but comfortable for in-the-eye use.

With conventional contact lens cleaners or disinfectants, including multipurpose solutions, lens wearers must typically manually rub the contact lenses, usually between a finger and the palm, or between the fingers, during treatment of the contact lenses. The necessity for the daily "rubbing" of contact lenses adds to the time and effort involved in the daily care of contact lenses. Many contact lens wearers dislike having to perform such a regimen or consider it to be an inconvenience. Some wearers may be negligent in the proper "rubbing" regimen, which may result in contact lens discomfort and other problems. Sometimes rubbing, if performed too rigorously, which is particularly apt to occur with beginning lens wearers, may damage the lenses. This can be problematic when a replacement lens is not immediately available. Additionally, some wearers may be negligent in the proper "rinsing" regimen, which may result in contact lens discomfort and other problems.

Contact lens solutions that qualify as a "Chemical Disinfecting Solution" do not require rubbing to meet biocidal performance criteria for destroying representative bacteria and fungi as established by the U.S. Food and Drug Administration (FDA) under the Premarket Notification (510K) Guidance Document for Contact Lens Care Products, May 1, 1997. In contrast, contact lens solutions that qualify as a "Chemical Disinfecting System" do require a rubbing regimen to pass biocidal performance criteria. Traditionally, multipurpose solutions used for disinfecting and wetting or for disinfecting, cleaning and wetting qualify as Chemical Disinfecting Systems, but not as Chemical Disinfecting Solutions.

Traditional contact lens solutions may depend on the rubbing regimen, not only for efficacious disinfection, but also for efficacious cleaning. Thus, in order to develop a contact lens care solution that would provide efficacious cleaning without a rubbing or rinsing regimen, as opposed to "rub and rinse" and/or "no rub with rinse" regimens for cleaning, would require improved cleaning efficacy while still being sufficiently gentle for in-the-eye use.

G.B. Patent No. 1,432,345 proposes contact lens disinfecting compositions containing a polymeric biguanide and a mixed phosphate buffer. The compositions proposed by this patent, however, have corneal staining values of 17 percent or more, far above that which is desirable for patient acceptability.

U.S. Patent No. 4,758,595 proposes a contact lens solution containing a poly(aminopropyl biguanide) (PAPB), also known as poly(hexamethylene biguanide) (PHMB), having enhanced efficacy when combined with a borate buffer. The proposed disinfecting and preservative solutions are said to possess bactericidal and fungicidal activity at low concentrations coupled and are further said to possess low toxicity when used with soft-type contact lenses. U.S. Patent No. 4,758,595 indicates that compositions containing PHMB and borate have been commercialized in various products including multi-purpose solutions, at levels of about 1 ppm or less, for use with soft contact lenses.

U.S. Patent No. 4,820,352 proposes compositions for cleaning and conditioning contact lenses where the primary cleaning agent is a specific class of polyethyleneoxy-polypropyleneoxy block copolymer adduct of ethylene diamine (also known as poloxamine). The compositions proposed are said to be sufficiently nonirritating and it is suggested that a contact lens treated with such a solution can be inserted directly in the eye.

U.S. Patent No. 5,209,865 proposes a conditioning solution for contact lenses that comprises a combination of a poloxamine and a poloxamer surfactant each having an HLB (hydrophilic-lipophilic balance) of seven or below. It is suggested that a solution that forms a uniform hydrophilic film on a lens surface may be produced wherein proteins may have very little affinity.

U.S. Patent No. 5,604,189 and U.S. Patent No. 5,773,396 propose a composition for cleaning and wetting contact lenses comprising (i) a non-amine polyethyleneoxy-containing compound having an HLB of at least about 18, (ii) a surface active agent having cleaning activity for contact lens deposits that may have an HLB of less than 18, and (iii) a wetting agent. In another approach, it is proposed that Tyloxapol may be employed as a conventional surface active agent in a multipurpose solution, the agent said to have cleaning activity for contact-lens deposits and an HLB less than 18.

U.S. Patent No. 6,143,244 proposes a method for treating contact lenses and compositions for such use. The proposed method includes the use of an aqueous biguanide-containing disinfecting solution including a buffer system comprising a mixture of a phosphate and a borate buffer. Also proposed are methods and compositions for simultaneously cleaning and disinfecting contact lenses.

U.S. Patent No. 6,790,816 proposes a no rub cleaning and disinfecting solution that is said to possess an effective amount of an antimicrobial and an osmolyte.

U.S. Patent Publication No. 2002/0141899 proposes a composition for disinfecting a contact lens that employs hydrogen peroxide and a surfactant.

U.S. Patent Publication No. 2003/0118472 proposes a system and method for disinfecting and cleaning contact lenses. The system proposed involves the use of an iodide salt and hydrogen peroxide. A no rub-no rinse regimen is also proposed.

U.S. Patent Publication No. 2003/0153475 proposes a method for cleaning contact lenses that employs tromethamine and suggests that manual rubbing is unnecessary. Also proposed is a method that does not include a manual rinse step.

U.S. Patent Publication No. 2004/0185028 proposes a composition comprising one or more ethanolamine buffers and one or more antimicrobial agents for the purpose of disinfecting contact lenses.

U.S. Application Serial Nos. 10/926,514 and 10/926,504 each propose lens care solutions that utilize trialkanolamine alkoxylate; N, N, N¹, N¹-tetrakis(hydroxyalkyl)diamine; and N, N, N¹, N¹-tetrakis (hydroxyalkoxy)-diamine based buffers.

U.S. Patent Publication No. 2005/119221 describes the use of a composition of one or more cationic polysaccharides in combination with one or more antimicrobial agents to disinfect contact lenses and preserve ophthalmic lens compositions.

U.S. Patent Publication No. 2005/261148 describes the use of one or more compositions of one or more disinfecting agents at a relatively high pH to disinfect contact lenses and preserve ophthalmic lens compositions.

WO 2004/112848 A1 describes contact lens cleaning and disinfecting compositions that reduce or eliminate wearer discomfort comprising a disinfecting amount of Alexidine. It is further described that the solutions are effective in removing protein and lipid tear film deposits on both hard and soft contact lenses.

It would be desirable to obtain a multipurpose contact lens solution that would provide increased cleaning efficacy for RGP lenses. It would likewise be desirable to obtain improved cleaning efficacy for RGP lenses while maintaining both biocidal efficacy of the solution and low order toxicity of the solution to eye tissue. It would further be desirable to provide a multipurpose contact lens solution possessing low order toxicity to eye tissue, such that after the solution is used to treat a contact lens, the lens can be subsequently placed on the eye without rinsing the solution from the lens. While still more challenging to develop, it would also be desirable to obtain a solution that exhibits both efficacious cleaning and disinfection of a contact lens, particularly RGP lenses, without requiring a rubbing or rinsing regimen, or at least not inherently or invariably requiring rubbing and rinsing, for acceptable performance. Such a solution would permit the direct placement of the contact lens on an eye following soaking in the solution and/or rinsing and rewetting with the solution.

### Summary of the Invention

The present invention includes a rigid gas permeable lens care solution. The lens care solution comprises a disinfecting amount of at least one biguanide antimicrobial agent and a straight chain polyether surfactant based upon PEO-PPO-PEO, PPO-PEO-PPO and combinations thereof. The lens care solution is capable of cleaning the rigid gas permeable lens in the absence of manually rubbing or rinsing the rigid gas permeable lens.

In one embodiment, the rigid gas permeable lens care has a straight chain polyether surfactant with an HLB value of less than or equal to 15 and an average molecular weight value of between about 5000 and 7000.

The rigid gas permeable lens care solution of one embodiment has a straight chain polyether surfactant with the chemical structure PEO₂₀PPO₇₀PEO₂₀.

In another embodiment, the rigid gas permeable lens care solution has a straight chain polyether surfactant that has a chemical structure PEO₃₇PPO₅₆PEO₃₇.

In one embodiment, there is a method of producing a rigid gas permeable lens care solution comprising the step of combining at least one biguanide antimicrobial agent with an effective amount of a straight chain polyether surfactant based upon PEO-PPO-PEO, PPO-PEO-PPO and combinations thereof. In the above method, the lens care solution is effective in cleaning the rigid gas permeable lens in the absence of manually rubbing or rinsing the rigid gas permeable lens. In another embodiment, the solution includes a straight chain polyether surfactant that has an HLB value of less than or equal to 15 and an average molecular weight value of between about 5000 and 7000.

In another embodiment, there is a method of using a lens care solution with a rigid gas permeable lens. The lens care solution comprising a disinfecting amount of at least one biguanide antimicrobial agent and a straight chain polyether surfactant based upon PEO-PPO-PEO, PPO-PEO-PPO and combinations thereof, the lens care solution being effective to clean the rigid gas permeable lens in the absence of manually rubbing or rinsing the rigid gas permeable lens. The method comprises the step of contacting the lens with the solution for a period of time suitable to reduce or eliminate a microbial burden on the lens and directly placing the lens on an eye.

In another embodiment, the method provides complete cleaning of the lens such that digital rubbing of the lens is not necessary to clean the lens. Preferably, the method is carried out in the absence of agitation, rubbing and rinsing. In one embodiment, the solution contains a straight chain polyether surfactant that has an HLB value of less than or equal to 15 and an average molecular weight value of between about 5000 and 7000.

### Detailed Description

The compositions and lens care solutions disclosed herein can be used with all contact lenses such as conventional hard and soft-type lenses, as well as rigid and soft gas permeable lenses. Such lenses include both hydrogel and non-hydrogel lenses, as well as silicone and fluorine-containing lenses. Of primary interest is the class of lenses known to those skilled in the art as rigid gas permeable (RGP) lenses, or more recently as gas permeable (GP) lenses. The compositions are also useful for disinfecting and cleaning medical devices, particularly those soiled with proteinaceous matter.

As may be appreciated by those skilled in the art, RGP lenses are normally of the silicone acrylate-type or the fluorosilicone-acrylate type. Such materials are often prepared from a siloxanyl methacrylate, such as 3-methacryloyloxypropyltris-(trimethylsiloxy)silane, a difunctional, polymerizable silicone macromonomer, one or more crosslinking agents, such as tetraethylene glycol dimethacrylate, one or more hydrophobic monomers, such as methyl methacrylate or hexafluoroisopropyl methacrylate, and one or more hydrophilic monomers, such as 2-hydroxyethyl methacrylate, N-vinylpyrrolidone and/or methacrylic acid. In contrast to soft, hydrogel lenses, where water content often ranges from 20% to 80%, an RGP lens generally has a water content of less than 1%.

The compositions are preferably placed in solution in sufficient concentration to destroy harmful microorganisms on the surface of a contact lens within the recommended minimum soaking time. The recommended minimum soaking time is one designed to be commercially acceptable to consumers and is typically included in the package instructions for use of the solution. The term "disinfecting solution" as used herein does not exclude the possibility that the solution may also be useful as a preserving solution, or that the disinfecting solution may be useful for other purposes such as daily cleaning, rinsing, and storage of contact lenses, depending on the particular formulation containing the subject compositions. Additionally, the compositions disclosed herein can be used in conjunction with other known disinfecting or preserving compounds, if desired.

The compositions disclosed herein, when employed in solution, are physiologically compatible. Specifically, the solutions are "ophthalmically safe" for use with a contact lens, meaning that a contact lens treated with a solution of the present invention is generally suitable and safe for direct placement on the eye, without rinsing. The solutions are safe and comfortable for daily contact with the eye via a contact lens that has been wetted with the solution. An ophthalmically safe solution has a tonicity and pH that is compatible with the eye and comprises materials, and amounts thereof, that are non-cytotoxic according to relevant International Standards Organization (ISO) standards and U.S. Food and Drug Administration (FDA) regulations. The solution should be sterile in that the absence of microbial contaminants in the product prior to release must be statistically demonstrated to the degree necessary for such products.

The compositions and lens care solutions disclosed herein comprise one or more biguanide antimicrobial agents for use as a disinfectant, and one or more surfactants. The one or more biguanide antimicrobial agents are present in a total amount of from approximately 0.000001 to approximately 5.0 percent, by weight, based on the total weight of the composition. The one or more biguanide antimicrobial agents are preferably present from about 0.00001 to about 5.0 weight percent, more preferably from about 0.00001 to about 1.0 weight percent and most preferably from about 0.0001 to about 1.0 weight percent. Suitable antimicrobial agents include, for example, but are not limited, to 1,1'-hexamethylenebis[5-(p-chlorophenyl)biguanide] (Chlorhexidine) or water soluble salts thereof, 1,1'-hexamethylenebis[5-(2-ethylhexyl)biguanide] (Alexidine) or water soluble salts thereof, poly(hexamethylene biguanide) or water soluble salts thereof, polyquaternium-1, ionene polymers and quaternary ammonium esters.

Suitable biguanides are described in U.S. Patent Nos. 3,428,576, 4,758,595 and 5,990,174 each incorporated by reference herein in its entirety. The preferred antimicrobial agents, based on their ready commercial availability, are poly(aminopropyl biguanide) (PAPB), also commonly referred to as poly(hexamethylene biguanide) (PHMB), and 1,1'-hexamethylenebis[5-(2-ethylhexyl)biguanide] (Alexidine).

In one preferred form, a combination of PHMB and Alexidine is used as the biguanide antimicrobial agent alone or in combination with another antimicrobial agent. Moreover, it has been discovered that the introduction of Alexidine decreases the required time for disinfection significantly and, advantageously, the compositions disclosed herein cause no burning and stinging when lenses soaked in same are inserted into the eye.

When formulated as a cleaning solution, the compositions disclosed herein include a total amount of from about 0.001 to about 6 percent, by weight, of one or more surfactants capable of providing advantages in terms of cleaning efficacy and comfort. As may be appreciated, surfactants for use herein should be soluble in the lens care solution, not become turbid, and should be nonirritating to eye tissues. Suitable surfactants include, for example, but are not limited to, water-soluble triblock copolymer polyethers based upon poly(ethylene oxide)-poly(propylene oxide)-poly(ethylene oxide), i.e., (PEO-PPO-PEO), or poly(propylene oxide)-poly(ethylene oxide)-poly(propylene oxide), i.e., (PPO-PEO-PPO), or combinations thereof. PEO-PPO-PEO and PPO-PEO-PPO surfactants are commercially available from BASF Corporation of Mt. Olive, New Jersey under the trademarks Pluronic^{®}, R-Pluronic^{®}, Tetronic^{®} and R-Tetronic^{®} and are further described in U.S. Patent No. 4,820,352, which is hereby incorporated by reference in its entirety.

Pluronic^{®} polyether surfactants are straight chain or linear polyether surfactants and belong to the class of materials generically referred to as "poloxamers." A linear polyether surfactant is considered to have no branched or graft junctions, but may include pendant functional groups. The straight chain polyether surfactants disclosed herein comprise one or more chains or polymeric components having oxyalkylene (-O-R-) repeating units, wherein R has 2 to 6 carbon atoms. Their solubility in water is generally good, but the properties of the individual block copolymers vary substantially. The nomenclature used for the block copolymers, and generally herein, is such that the first two figures, when multiplied by 100, represent the average molecular weight of the PO block, whilst the last figure, when multiplied by 10, represents the ethylene oxide content (% w/w) of the poloxamer.

Grades of commercially available poloxamer surfactants are available with number average molecular weights (average molecular weight) ranging from as low as 1650 to 27,000. The properties of each grade within the series vary depending on the percent of hydrophilic units poly(oxyethylene) and molecular weight of hydrophobic units poly(oxypropylene) in the adduct. While all members within the series exhibit wetting and detergency properties, it was discovered that only certain members are suitable for use in the cleaning and conditioning solutions disclosed herein, due to the wide variation in performance characteristics regulated by their hydrophilic-hydrophobic balance (HLB). The poloxamer surfactants found suitable are those capable of demonstrating maximum cleaning efficiency in dispersing both protein ahd lipid deposits at ambient and elevated temperatures at lowest solution concentration, without trade-offs in lens compatibility and toxicity levels, i.e. maintaining the lowest potential as an irritant to eye tissues.

Suitable straight chain polyether surfactants having an HLB value less than or equal to 15 include, for example, but are not limited to Pluronic^{®} L42 (BASF) having an HLB of 8 and average molecular weight of 1630; Pluronic^{®} L63 (BASF) having an HLB of 11 and average molecular weight of 2650; Pluronic^{®} L101 (BASF) having an HLB of 1 and average molecular weight of 3800; Pluronic^{®} P103 (BASF) having an HLB of 9 and average molecular weight of 4950; Pluronic^{®} P123 (BASF) having an HLB of 8 and average molecular weight of 5750; Pluronic^{®} L122 (BASF) having an HLB of 4 and average molecular weight of 5000; Pluronic^{®} L121 (BASF) having an HLB of 1 and average molecular weight of 4400; Pluronic^{®} L92 (BASF) having an HLB of 6 and average molecular weight of 3650; Pluronic^{®} L81 (BASF) having an HLB of 2 and average molecular weight of 2750; Pluronic^{®} L72 (BASF) having an HLB of 7 and average molecular weight of 2750; Pluronic^{®} L62 (BASF) having an HLB of 7 and average molecular weight of 2500; Pluronic^{®} L61 (BASF) having an HLB of 3 and average molecular weight of 2000; Pluronic^{®} L31 (BASF) having an HLB of 5 and average molecular weight of 1100; and Pluronic^{®} P105 (BASF) having an HLB of 15 and average molecular weight of 6500.

A particularly preferred straight chain polyether surfactant having an HLB value less than or equal to 15 is sold by BASF Corporation as Pluronic^{®} P123. Pluronic^{®} P123 may be characterized as PEO₂₀PPO₇₀PEO₂₀ and, as indicated above possesses an HLB of 8 and average molecular weight of 5750. Another particularly preferred straight chain polyether surfactant having an HLB value less than or equal to 15 is sold by BASF Corporation as Pluronic^{®} 105. Pluronic^{®} P105 may be characterized as PEO₃₇PPO₅₆PEO₃₇, and, as indicated above possesses an HLB of 15 and average molecular weight of 6500. As may be appreciated by those skilled in the art, it is surprising that the subject compositions exhibit excellent disinfecting and/or preservative characteristics without the aid of borate, contrary to the teachings of U.S. Patent Number 4,758,595.

Branched chain polyether surfactants may be added to the compositions disclosed herein. Poloxamines, such as the Tetronic^{®} polyether surfactants are branched chain surfactants are particularly preferred. A branched chain polyether surfactant is considered to be a star-type polymer, such as a tetra-polyether substituted derivative of ethylenediamine, or the like. The branched chain polyether surfactants disclosed herein comprise one or more chains or polymeric components having oxyalkylene (-O-R-) repeats units wherein R has 2 to 6 carbon atoms. Representative polyether surfactants comprise block polymers of two or more different kinds of oxyalkylene repeating units, the ratio of which serves to determine the HLB of the surfactant.

Suitable branched and straight chain polyether surfactants have an HLB value greater than or equal to 18 and include, for example, but are not limited to Tetronic^{®} 707 (BASF) having an HLB of 27 and average molecular weight of 12200; Tetronic^{®} 908 (BASF) having an HLB of 31 and average molecular weight of 25000; Tetronic^{®} 909 (BASF) having an HLB of 32 and average molecular weight of 30000; Tetronic^{®} 1107 (BASF) having an HLB of 24 and average molecular weight of 15000; Tetronic^{®} 1307 (BASF) having an HLB of 24 and average molecular weight of 18000; and Tetronic^{®} 1508 (BASF) having an HLB of 27 and average molecular weight of 30000. Particularly preferred is Tetronic^{®} 909 (BASF) having an HLB of 32 and average molecular weight of 30000 and Tetronic^{®} 1107 (BASF) having an HLB of 24 and average molecular weight of 15000.

As indicated, the HLB of a surfactant is known to be a major factor in determining the emulsification characteristics of a polyether surfactant. In general, surfactants with lower HLB values are more lipophilic, while surfactants with higher HLB values are more hydrophilic. The HLB values of various poloxamines and poloxamers are provided by BASF Wyandotte Corp., Wyandotte, Mich.

Additional straight chain poly(ethylene oxide-propylene oxide-ethylene oxide) (PEO-PPO-PEO) block copolymers having an HLB value of greater than or equal to 18 may be added to the aqueous composition of the invention. An example of a suitable material is Pluronic^{®} F127 (BASF) having an HLB of 18-23 and average molecular weight of 12600.

In one embodiment of the present invention, Pluronic^{®} P123 is used alone or in combination with other surfactants. In another embodiment of the present invention, Pluronic^{®} P105 is used alone or in combination with other surfactants. Both Pluronic^{®} P123 and Pluronic^{®} P105 have been found to effectively remove lipid deposits from the surface of RGP lenses.

Optionally included may be one or more water-soluble viscosity agents. Because of the demulcent effect of viscosity agents, a tendency is evidenced to enhance the lens wearer's comfort by means of providing a film on the lens surface, cushioning the impact against the eye. Suitable viscosity agents include, for example, but are not limited, to water-soluble cellulose polymers such as hydroxyethylcellulose, hydroxypropylcellulose or hydroxypropylmethylcellulose, guar, hydroxyethylguar, hydroxypropylguar, hydroxypropylmethylguar, poly(N,N-dimethylacrylamide), poly(N-vinylpyrrolidone), poly(vinyl alcohol), poly(ethylene glycol), poly(ethylene oxide) and the like. Viscosity agents may be employed in amounts ranging from about 0.01 to about 4.0 weight percent or less.

The compositions disclosed herein may likewise include one or more buffers, or a buffering system to adjust the final pH of the solution. Suitable buffers include, for example, but are not limited to, citrate buffers, phosphate buffers, borate buffers, tris(hydroxymethyl)aminomethane (Tris) buffers, sodium bicarbonate, as well as the Good Buffers, including, but not limited to, N,N'-bis(2-hydroxyethyl)glycine (BICINE), 2-[bis(2-hydroxyethyl)amino]-2-(hydroxymethyl)-1,3-propanediol (BISTRIS), 2-(cyclohexylamino)ethane-2-sulfonic acid (CHES), N-(2-hydroxyethyl)ethylenediamine-N,N',N'-triacetic acid (EDTA-OH), N-(2-hydroxypropyl)ethylenediamine -N, N',N'-triacetic acid (propyl-EDTA-OH), N-2-(hydroxyethyl)- piperazine-N'-2-ethanesulfonic acid (HEPES), N-(2-hydroxyethyl)- piperazine-N'-3-propanesulfonic acid (HEPPS), morpholinoethanesulfonic acid (MES), morpholinopropanesulfonic acid (MOPS), piperazine-N,N'-bis(2-ethanesulfonic acid) (PIPES), N-tris(hydroxymethyl)methyl-2-aminoethanesulfonic acid (TES), N-tris(hydroxymethyl)methyl-3-aminopropanesulfonic acid (TAPS), and N-tris(hydroxymethyl)- methylglycine (TRICINE), and combinations thereof. The well-known Good Buffers (Good N.E. et al., (1966) Biochemistry, 5, 467-477) are non-toxic to cells, not absorbed through cell membranes and feature pKa values at or near physiological pH. The pH of the lens care solutions disclosed herein is preferably maintained within the range of 5.0 to 8.0, more preferably about 6.0 to 8.0, most preferably about 6.5 to 7.8.

One or more tonicity agents (osmotic agents) may likewise be included to approximate the osmotic pressure of normal lachrymal fluids, which is equivalent to a 0.9 percent solution of sodium chloride or 2.5 percent glycerin solution. Examples of suitable tonicity agents include, but are not limited to, zinc chloride, sodium chloride, potassium chloride, dextrose, mannose, glycerin, propylene glycol, calcium chloride and magnesium chloride. These agents are typically used individually in amounts ranging from about 0.01 to about 2.5 percent w/v and preferably from about 0.2 to about 1.5 percent w/v. It is preferred that the tonicity agent be employed in an amount to provide a final osmotic value of 200 to 450 mOsm/kg and more preferably between about 205 to about 350 mOsm/kg, and most preferably between about 210 to about 320 mOsm/kg.

The compositions disclosed herein may likewise include one or more sequestering agents to bind metal ions, which in the case of ophthalmic solutions, might otherwise react with protein deposits and collect on contact lenses. Suitable sequestering agents include, for example, but are not limited to, ethylenediaminetetraacetic acid (EDTA) and its salts, gluconic acid, citric acid, tartaric acid and their salts, such as sodium salts. Sequestering agents are preferably used in amounts ranging from about 0.01 to about 0.2 weight percent.

One or more cationic polysaccharides may also be optionally included. When utilized, the one or more cationic polysaccharides are present in a total amount of from approximately 0.001 to approximately 0.5 percent, by weight, based on the total weight of the composition and more preferably from about 0.005 to about 0.05 percent, by weight. Suitable cationic polysaccharides for use in the compositions disclosed herein include, for example, but are not limited to variations of Polyquaternium-10 such as for example but not limited to Polymer JR 125^{™} (Dow Chemical Company of Midland, Michigan) having a 2 percent solution viscosity of 75-125 cPs and 1.5 to 2.2 percent nitrogen, Polymer JR 400^{™} (Dow Chemical Company) having a 2 percent solution viscosity of 300 to 500 cPs and 1.5 to 2.2 percent nitrogen, Polymer JR 30 M^{™} (Dow Chemical Company) having a 1 percent solution viscosity of 1,000 to 2,500 cPs and 1.5 to 2.2 percent nitrogen, Polymer LR 400^{™} (Dow Chemical Company) having a 2 percent solution viscosity of 300 to 500 cPs and 0.8 to 1.1 percent nitrogen, Polymer LR 30M^{™} (Dow Chemical Company) having a 1 percent solution viscosity of 1,250 to 2,250 cPs and 0.8 to 1.1 percent nitrogen, and Polymer LK^{™} (Dow Chemical Company) having a 2 percent solution viscosity of 300 to 500 cPs and 0.8 to 1.1 percent nitrogen. The preferred cationic polysaccharide for use herein is Polymer JR 125^{™} or Polymer JR 400^{™}.

The compositions disclosed herein may likewise include one or more water-soluble carbohydrates. Such carbohydrates are present in the subject compositions in a total amount of from approximately 0.01 to approximately 10.0 percent, by weight, based on the total weight of the composition, but more preferably from about 0.05 to about 5.0 percent, by weight. Suitable carbohydrates for use herein include, for example, but are not limited to, monosaccharides, disaccharides, oligosaccharides and polysaccharides. Suitable monosaccharides include, for example, but are not limited to, allose, altrose, glucose, mannose, gulose, idose, galactose, talose, ribose, arabinose, xylose and lyxose. Examples of suitable disaccharides are sucrose and trehalose. Suitable oligosaccharides, composed of two to eight units of monosaccharide, and polysaccharides, composed of more than eight units of monosaccharide, include, for example, but are not limited to, agar, agarose, guar gum, hydroxypropylguar, hydroxypropylmethylguar, hydroxyethylguar, carboxymethylguar, gum arabic, dextran, locust bean, alginates, asafetida, gum benzoin, carragreenans, carob, colophone, galbanum, gum damar, gum cassia, hydroxyethylcelluose, hydroxypropylcellulose, hydroxypropylmethylcellulose, methylcellulose, carboxymethylcellulose, gum chicle, gum elemi, gum gambodge, gum rosin, gum sandarac, gum tara, gum terpentine, gum tragacanth, xanthan gum, gum yucca, pectin, gellen gum, hyaluronic acid, chondroitin sulfate, gum ghatti, gum guaiac, gum guaiac, gum guarana, gum guttae, gum karaya, gum konjac, gum mastix, gum myrrh and gum olibanum.

The subject compositions may likewise include a wetting agent, to facilitate the composition wetting the surface of a contact lens. Within the art, the term "humectant" is also commonly used to describe these materials. A first class of wetting agents is polymeric wetting agents. Examples of suitable wetting agents include, for example, but are not limited to, poly(vinyl alcohol) (PVA), poly(N-vinylpyrrolidone) (PVP), water-soluble cellulose derivatives such as hydroxypropylmethylcellulose and poly(ethylene glycol). Water-soluble cellulose derivatives and PVA may be used to also increase viscosity of the composition, and offer this advantage, if desired. Specific cellulose derivatives include for example but are not limited to hydroxypropylmethylcellulose, carboxymethylcellulose, methylcellulose, hydroxyethylcellulose, and cationic cellulose derivatives. As disclosed in U.S. Patent No. 6,274,133, cationic cellulosic polymers also help prevent accumulation of lipids and proteins on a hydrophilic lens surface. Such cationic cellulosic polymers include, for example but are not limited to, the water soluble polymers commercially available under the CTFA (Cosmetic, Toiletry, and Fragrance Association) designation Polyquatemium-10, including the cationic cellulosic polymers available under the trade name UCARE^{®} Polymers from Amerchol Corp., Edison, New Jersey. Generally, these cationic cellulose polymers contain quatemized N,N-dimethylamino groups along the cellulosic polymer chain.

Another suitable class of wetting agents is non-polymeric wetting agents. Examples include glycerin, propylene glycol, and other non-polymeric diols and glycols.

The specific quantities of wetting agents used herein will vary depending upon the application. However, the wetting agents will typically be included in an amount from about 0.01 to about 5 weight percent, preferably from about 0.1 to about 2 weight percent.

The subject compositions are described in still greater detail in the examples that follow.

In the examples below, certain chemical ingredients are identified by the following abbreviations.
HAP: Hydroxyalkylphosphorate in phosphate-buffered saline (PBS) with 0.5 U of aprotinin per ml-0.05% human serum albumin-3 mM D-glucose;
Polymer JR^{®}: cationic polysaccharide, polyquaternium-10; and Alexidine 2HCl: quaternary ammonium salt, 1,1'-hexamethylenebis[5-(2-ethylhexyl)biguanide]

### Examples 1-14 - Biocidal Stand-Alone Testing of Compositions with PHMB HCl and Pluronic^{®} P123

The antimicrobial efficacy of solutions prepared with Pluronic^{®} P123 and PHMB HCl for the chemical disinfection of contact lenses, using 10% organic soil, is evaluated. The solution ingredients are set forth in Tables 1 to 14 below.

Microbial challenge inoculums are prepared using *Pseudomonas aeruginosa* (ATCC 9027), *Staphylococcus aureus* (ATCC 6538), *Serratia marcescens* (ATCC 13880), *Candida albicans* (ATCC 10231) and *Fusarium solani* (ATCC 36031). The test organisms are cultured on appropriate agar and the cultures are harvested using sterile Dulbecco's Phosphate Buffered Saline plus 0.05 percent weight/volume polysorbate 80 (DPBST) or a suitable diluent and transferred to a suitable vessel. Spore suspensions are filtered through sterile glass wool to remove hyphal fragments. *Serratia marcescens*, as appropriate, is filtered through a 1.2 micron filter to clarify the suspension. After harvesting, the suspension is centrifuged at no more than 5000 x g for a maximum of 30 minutes at 20 to 25 degrees Celsius. The supernatent is poured off and resuspended in DPBST or other suitable diluent. The suspension is centrifuged a second time, and resuspended in DPBST or other suitable diluent.

All challenge bacterial and fungal cell suspensions are adjusted with DPBST or other suitable diluent to 1 x 10⁷ to 1 x 10⁸ cfu/mL. The appropriate cell concentration may be estimated by measuring the turbidity of the suspension, for example, using a spectrophotometer at a preselected wavelength, for example 490 nm. One tube is prepared containing a minimum of 10 mL of test solution per challenge organism. Each tube of the solution to be tested is inoculated with a suspension of the test organism sufficient to provide a final count of 1 x 10⁵ to 1 x 10⁶ cfu/mL, the volume of the inoculum not exceeding 1 percent of the sample volume. Dispersion of the inoculum is ensured by vortexing the sample for at least 15 seconds. The inoculated product was stored at 20 to 25 degrees Celsius. Aliquots in the amount of 1.0 mL were taken of the inoculated product for determination of viable counts after certain time periods of disinfection. The time points for the bacteria are, for example, 1, 2, 3, 4 and 24 hours, when the proposed regimen soaking time is four hours for 25%, 50%, 75%, 100% and 400% soaking time. The suspension is mixed well by vortexing vigorously for at least 5 seconds. The 1.0 mL aliquots removed at the specified time intervals are subjected to a suitable series of decimal dilutions in validated neutralizing media. The suspensions are mixed vigorously and incubated for a suitable period of time to allow for neutralization of the microbial agent.

In order to demonstrate the suitability of the medium used for growth of test organisms and to provide an estimation of the initial inoculum concentration, inoculum controls are made by dispersing an identical aliquot of the inoculum into a suitable diluent, for example DPBST, using the same volume of diluent used to suspend the organism listed above. Following inoculation in a validated neutralizing broth and incubation for an appropriate period of time, the inoculum control must be between 1.0 x 10⁵ to 1.0 x 10⁶ cfu/mL.

The viable count of organisms is determined in appropriate dilutions by preparation of triplicate plates of tryptic soy agar (TSA) for bacteria and Sabouraud dextrose agar (SDA) for mold and yeast. The bacterial recovery plates are incubated at 30 to 35 degrees Celsius for two to four days. The yeast recovery plates are incubated at 20 to 30 degrees Celsius for two to four days. The mold recovery plates are incubated at 20 to 25 degrees Celsius for three to seven days. The average number of colony forming units is determined on countable plates. Countable plates refer to 30 to 300 cfu/plates for bacteria and yeast, and 8 to 80 cfu/plates for mold except when colonies are observed only for the 10⁰ or 10⁻¹ dilution plates. The microbial reduction are calculated at the specified time points and recorded as set forth below in Tables 1 to 14 below.

The biocidal results in Examples 1 to 14 meet the ISO Stand Alone Procedure for Contact Lens Disinfecting Products requirements at 1 and 4 hours. The results also show that the presence of 10% organic soil will not decrease the biocidal efficacies against the five challenge organisms using the formulations disclosed herein.

The organic soil is a mixture of heat shocked *Saccharomyces cerevisiae* [1 X 10⁷] mixed, per ml, with Serum Bovine Albumin. To obtain a 10% concentration, the mixture is diluted 1 part to 9 parts with PBS.

**TABLE 1**

| Ingredients Example 1 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.088 |
| Polymer JR | | 0.020 |
| Pluronic^{®} F127 | | 2.500 |
| Pluronic^{®} P123 | | 0.500 |
| PHMB HCl | | 3.0 ppm |
| pH = 6.8- 7.2 | | |
| Osmolality (mOsmo/Kg) = 240-280 | | |
| *S. aureus* | 1 HR | 2.6 |
| | 4 HR | 3.6 |
| *P. aeruginosa* | 1 HR | >4.9 |
| | 4 HR | >4.9 |
| *S. marcescens* | 1 HR | >4.9 |
| | 4 HR | >4.9 |
| *C. albicans* | 1 HR | NA |
| | 4 HR | NA |
| *F. solani* | 1 HR | NA |
| | 4 HR | NA |

**TABLE 2**

| Ingredients Example 2 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.088 |
| Polymer JR | | 0.020 |
| Pluronic^{®} F127 | | 2500 |
| Pluronic^{®} P123 | | 0.500 |
| PHMB HCl | | 5.0 ppm |
| pH = 6.8- 7.2 | | |
| Osmolality (mOsmo/Kg) = 240-280 | | |
| *S. aureus* | 1 HR | 3.5 |
| | 4 HR | 4.7 |
| *P. aeruginosa* | 1 HR | >4.9 |
| | 4 HR | >4.9 |
| *S. marcescens* | 1 HR | >4.9 |
| | 4 HR | >4.9 |
| *C. albicans* | 1 HR | NA |
| | 4 HR | NA |
| *F. solani* | 1 HR | NA |
| | 4 HR | NA |

**TABLE 3**

| Ingredients Example 3 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.088 |
| Polymer JR | | 0.020 |
| Pluronic^{®} F127 | | 5.500 |
| Pluronic^{®} P123 | | 0.500 |
| HPMC | | 0.200 |
| PHMB HCl | | 5.0 ppm |
| pH = 6.8- 7.2 | | |
| Osmolality (mOsmo/Kg) = 240-280 | | |
| *S. aureus* | 1 HR | 2.7 |
| | 4 HR | 3.7 |
| *P. aeruginosa* | 1 HR | >4.9 |
| | 4 HR | >4.9 |
| *S. marcescens* | 1 HR | >4.9 |
| | 4 HR | >4.9 |
| *C. albicans* | 1 HR | NA |
| | 4 HR | NA |
| *F. solani* | 1 HR | NA |
| | 4 HR | NA |

**TABLE 4**

| Ingredients Example 4 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.088 |
| Polymer JR | | 0.020 |
| Pluronic^{®} F127 | | 2.750 |
| Pluronic^{®} P123 | | 0.250 |
| PHMB HCl | | 5.0 ppm |
| pH = 6.8- 7.2 | | |
| Osmolality (mOsmo/Kg) = 260-280 | | |
| *S. aureus* | 1 HR | 2.0 |
| | 4 HR | 3.2 |
| *P. aeruginosa* | 1 HR | 3.3 |
| | 4 HR | >4.8 |
| *S. marcescens* | 1 HR | 3.3 |
| | 4 HR | 3.9 |
| *C. albicans* | 1 HR | NA |
| | 4 HR | NA |
| *F. solani* | 1 HR | NA |
| | 4 HR | NA |

**TABLE 5**

| Ingredients Example 5 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.020 |
| Pluronic^{®} F127 | | 3.000 |
| Pluronic^{®} P123 | | 0.250 |
| HPMC | | 0.300 |
| PHMB HCl | | 5.0 ppm |
| pH = 6.8- 7.2 | | |
| Osmolality (mOsmo/Kg) = 260-280 | | |
| *S. aureus* | 1 HR | 2.0 |
| | 4 HR | 3.4 |
| *P. aeruginosa* | 1 HR | 4.1 |
| | 4 HR | >4.9 |
| *S. marcescens* | 1 HR | 2.2 |
| | 4 HR | 3.9 |
| *C. albicans* | 1 HR | NA |
| | 4 HR | NA |
| *F. solani* | 1 HR | NA |
| | 4 HR | NA |

**TABLE 6**

| Ingredients Example 6 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 3.000 |
| Pluronic^{®} P123 | | 0.250 |
| HPMC | | 0.300 |
| PHMB HCl | | 5.0 ppm |
| pH = 6.8- 7.2 | | |
| Osmo.(mOsmo/Kg) = 260-280 | | |
| *S. aureus* | 1 HR | 2.9 |
| | 4 HR | 3.7 |
| *P. aeruginosa* | 1 HR | 4.2 |
| | 4 HR | >4.9 |
| *S. marcescens* | 1 HR | 2.5 |
| | 4 HR | 3.8 |
| *C. albicans* | 1 HR | NA |
| | 4 HR | NA |
| *F. solani* | 1 HR | NA |
| | 4 HR | NA |

**TABLE 7**

| Ingredients Example 7 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.020 |
| Pluronic^{®} F127 | | 2.500 |
| Pluronic^{®} P123 | | 0.100 |
| HPMC | | 0.200 |
| PHMB HCl | | 5.0 ppm |
| pH = 6.8- 7.2 | | |
| Osmolality (mOsmo/Kg) = 240-280 | | |
| *S. aureus* | 1 HR | 1.9 |
| | 4 HR | 3.0 |
| *P. aeruginosa* | 1 HR | 4.5 |
| | 4 HR | 4.5 |
| *S. marcescens* | 1 HR | 2.6 |
| | 4 HR | 3.9 |
| *C. albicans* | 1 HR | NA |
| | 4 HR | NA |
| *F. solani* | 1 HR | NA |
| | 4 HR | NA |

**TABLE 8**

| Ingredients Example 8 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.250 |
| HPMC | | 0.200 |
| PHMB HCl | | 5.0 ppm |
| pH = 6.8- 7.2 | | |
| Osmolality (mOsmo/Kg) = 240-280 | | |
| *S. aureus* | 1 HR | 4.9 |
| | 4 HR | >4.9 |
| *P. aeruginosa* | 1 HR | >4.9 |
| | 4 HR | >4.9 |
| *S. marcescens* | 1 HR | 4.7 |
| | 4 HR | >4.9 |
| *C. albicans* | 1 HR | NA |
| | 4 HR | NA |
| *F. solani* | 1 HR | NA |
| | 4 HR | NA |

**TABLE 9**

| Ingredients Example 9 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.020 |
| Pluronic^{®} F127 | | 2.500 |
| Pluronic^{®} P123 | | 0.100 |
| HPMC | | 0.300 |
| PHMB HCl | | 5.0 ppm |
| pH = 6.8- 7.2 | | |
| Osmolality (mOsmo/Kg) = 240-280 | | |
| *S. aureus* | 1 HR | 4.7 |
| | 4 HR | >4.9 |
| *P. aeruginosa* | 1 HR | >4.9 |
| | 4 HR | >4.9 |
| *S. marcescens* | 1 HR | 4.9 |
| | 4 HR | >4.9 |
| *C. albicans* | 1 HR | NA |
| | 4 HR | NA |
| *F. solani* | 1 HR | NA |
| | 4 HR | NA |

**TABLE 10**

| Ingredients Example 10 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.020 |
| Pluronic^{®} F127 | | 2.500 |
| Pluronic^{®} P123 | | 0.100 |
| HPMC | | 0.400 |
| PHMB HCl | | 5.0 ppm |
| pH = 6.8- 7.2 | | |
| Osmolality (mOsmo/Kg) = 240-280 | | |
| *S. aureus* | 1 HR | 3.3 |
| | 4 HR | 4.5 |
| *P. aeruginosa* | 1 HR | 3.9 |
| | 4 HR | >4.9 |
| *S. marcescens* | 1 HR | 3.5 |
| | 4 HR | >4.1 |
| *C. albicans* | 1 HR | NA |
| | 4 HR | NA |
| *F. solani* | 1 HR | NA |
| | 4 HR | NA |

**TABLE 11**

| Ingredients Example 11 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.020 |
| Pluronic^{®} F127 | | 2.500 |
| Pluronic^{®} P123 | | 0.250 |
| HPMC | | 0.400 |
| PHMB HCl | | 5.0 ppm |
| pH = 6.8- 7.2 | | |
| Osmolality (mOsmo/Kg) = 240-280 | | |
| *S. aureus* | 1 HR | 3.7 |
| | 4 HR | 4.8 |
| *P. aeruginosa* | 1 HR | 4.6 |
| | 4 HR | 4.9 |
| *S. marcescens* | 1 HR | 3.4 |
| | 4 HR | 4.1 |
| *C. albicans* | 1 HR | NA |
| | 4 HR | NA |
| *F. solani* | 1 HR | NA |
| | 4 HR | NA |

**TABLE 12**

| Ingredients Example 12 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.020 |
| Pluronic^{®} F127 | | 2.500 |
| Pluronic^{®} P123 | | 0.100 |
| HPMC | | 0.100 |
| PHMB HCl | | 5.0 ppm |
| pH = 6.8- 7.2 | | |
| Osmolality (mOsmo/Kg) = 240-280 | | |
| *S. aureus* | 1 HR | 4.5 |
| | 4 HR | >4.8 |
| *P. aeruginosa* | 1 HR | >4.7 |
| | 4 HR | >4.7 |
| *S. marcescens* | 1 HR | >4.9 |
| | 4 HR | >4.9 |
| *C. albicans* | 1 HR | >4.7 |
| | 4 HR | >4.7 |
| *F. solani* | 1 HR | 2.8 |
| | 4 HR | 3.3 |

**TABLE 13**

| Ingredients Example 13 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.020 |
| Pluronic^{®} F127 | | 2.500 |
| Pluronic^{®} P123 | | 0.250 |
| HPMC | | 0.100 |
| PHMB HCl | | 5.0 ppm |
| pH = 6.8 - 7.2 | | |
| Osmolality (mOsmo/Kg) = 240 - 280 | | |
| *S. aureus* | 1 HR | >4.8 |
| | 4 HR | >4.8 |
| *P. aeruginosa* | 1 HR | >4.7 |
| | 4 HR | >4.7 |
| *S. marcescens* | 1 HR | >4.9 |
| | 4 HR | >4.9 |
| *C. albicans* | 1 HR | >4.7 |
| | 4 HR | >4.7 |
| *F. solani* | 1 HR | 4.4 |
| | 4 HR | 4.4 |

**TABLE 14**

| Ingredients Example 14 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.020 |
| Pluronic^{®} F127 | | 2.500 |
| Pluronic^{®} P123 | | 0.250 |
| PHMB HCl | | 5.0 ppm |
| pH = 6.8- 7.2 | | |
| Osmolality (mOsmo/Kg) = 240-280 | | |
| *S. aureus* | 1 HR | 3.6 |
| | 4 HR | 3.4 |
| *P. aeruginosa* | 1 HR | 4.7 |
| | 4 HR | >4.7 |
| *S. marcescens* | 1 HR | >4.9 |
| | 4 HR | >4.9 |
| *C. albicans* | 1 HR | 4.7 |
| | 4 HR | >4.7 |
| *F. solani* | 1 HR | 3.2 |
| | 4 HR | 4.0 |

### Examples 15-32 - Biocidal Stand-Alone Testing of Compositions with PHMB HCl; Alexidine 2 HCl: and Pluronic^{®} P123

Tests are conducted to study the microbiocidal efficacy of solutions prepared according to the present invention with Pluronic^{®} P123 and a combination of PHMB HCl and Alexidine 2HCl using 10%, 50% and 100% organic soil. The test solutions are identified below in Tables 15 to 32. The antimicrobial efficacy of each of the solutions for the chemical disinfection of contact lenses is evaluated according to the procedures as set forth in Examples 1 to 14.

The microbial reduction using 10% organic soil are calculated at the specified time points and recorded as set forth below in Tables 15 to 28 below. The microbial reduction using 100% organic soil are calculated at the specified time points and recorded as set forth below in Tables 29 and 30 below. The microbial reduction using 50% organic soil are calculated at the specified time points and recorded as set forth below in Tables 31 and 32 below.

**TABLE 15**

| Ingredients Example 15 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.088 |
| Polymer JR | | 0.020 |
| Pluronic^{®} F127 | | 2.750 |
| Pluronic^{®} P123 | | 0.250 |
| HPMC | | 0.400 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 2.7 |
| | 4 HR | 3.5 |
| *P. aeruginosa* | 1 HR | 3.2 |
| | 4 HR | 3.9 |
| *S. marcescens* | 1 HR | 3.8 |
| | 4 HR | 4.1 |
| *C. albicans* | 1 HR | NA |
| | 4 HR | NA |
| *F. solani* | 1 HR | NA |
| | 4 HR | NA |

**TABLE 16**

| Ingredients Example 16 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.088 |
| Polymer JR | | 0.020 |
| Pluronic^{®} F127 | | 2.750 |
| Pluronic^{®} P123 | | 0.250 |
| HPMC | | 0.300 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 2.6 |
| | 4 HR | 3.6 |
| *P. aeruginosa* | 1 HR | 3.9 |
| | 4 HR | >4.9 |
| *S. marcescens* | 1 HR | 3.1 |
| | 4 HR | 4.6 |
| *C. albicans* | 1 HR | NA |
| | 4 HR | NA |
| *F. solani* | 1 HR | NA |
| | 4 HR | NA |

**TABLE 17**

| Ingredients Example 17 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.020 |
| Pluronic^{®} F127 | | 3.000 |
| Pluronic^{®} P123 | | 0.250 |
| HPMC | | 0.300 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 2.9 |
| | 4 HR | 3.7 |
| *P. aeruginosa* | 1 HR | 3.5 |
| | 4 HR | >4.9 |
| *S. marcescens* | 1 HR | 2.4 |
| | 4 HR | 4.6 |
| *C. albicans* | 1 HR | NA |
| | 4 HR | NA |
| *F. solani* | 1 HR | NA |
| | 4 HR | NA |

**TABLE 18**

| Ingredients Example 18 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 3.000 |
| Pluronic^{®} P123 | | 0.250 |
| HPMC | | 0.300 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 2.8 |
| | 4 HR | 3.5 |
| *P. aeruginosa* | 1 HR | 4.4 |
| | 4 HR | >4.9 |
| *S. marcescens* | 1 HR | 3.2 |
| | 4 HR | 4.6 |
| *C. albicans* | 1 HR | NA |
| | 4 HR | NA |
| *F. solani* | 1 HR | NA |
| | 4 HR | NA |

**TABLE 19**

| Ingredients Example 19 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.020 |
| Pluronic^{®} F127 | | 3.000 |
| Pluronic^{®} P123 | | 0.100 |
| HPMC | | 0.300 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 3.8 |
| | 4 HR | >4.8 |
| *P. aeruginosa* | 1 HR | 3.8 |
| | 4 HR | >4.9 |
| *S. marcescens* | 1 HR | 3.1 |
| | 4 HR | 3.8 |
| *C. albicans* | 1 HR | NA |
| | 4 HR | NA |
| *F. solani* | 1 HR | NA |
| | 4 HR | NA |

**TABLE 20**

| Ingredients Example 20 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.250 |
| HPMC | | 0.200 |
| PHMB HCl | | 4.0 ppm |
| Alexidine 2HCL | | 4.0 ppm |
| pH = 6.8- 7.2 | | |
| Osmolality (mOsmo/Kg) = 240-280 | | |
| *S. aureus* | 1 HR | 4.0 |
| | 4 HR | >4.9 |
| *P. aeruginosa* | 1 HR | >4.9 |
| | 4 HR | >4.9 |
| *S. marcescens* | 1 HR | 4.5 |
| | 4 HR | >4.9 |
| *C. albicans* | 1 HR | NA |
| | 4 HR | NA |
| *F. solani* | 1 HR | NA |
| | 4 HR | NA |

**TABLE 21**

| Ingredients Example 21 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.250 |
| HPMC | | 0.200 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 4.9 |
| | 4 HR | >4.9 |
| *P. aeruginosa* | 1 HR | >4.9 |
| | 4 HR | >4.9 |
| *S. marcescens* | 1 HR | 4.9 |
| | 4 HR | >4.9 |
| *C. albicans* | 1 HR | NA |
| | 4 HR | NA |
| *F. solani* | 1 HR | NA |
| | 4 HR | NA |

**TABLE 22**

| Ingredients Example 22 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.100 |
| HPMC | | 0.300 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | >4.9 |
| | 4 HR | >4.9 |
| *P. aeruginosa* | 1 HR | 4.7 |
| | 4 HR | >4.9 |
| *S. marcescens* | 1 HR | >4.9 |
| | 4 HR | >4.9 |
| *C. albicans* | 1 HR | NA |
| | 4 HR | NA |
| *F. solani* | 1 HR | NA |
| | 4 HR | NA |

**TABLE 23**

| Ingredients Example 23 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.250 |
| HPMC | | 0.300 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 2.9 |
| | 4 HR | 4.4 |
| *P. aeruginosa* | 1 HR | >4.9 |
| | 4 HR | >4.9 |
| *S. marcescens* | 1 HR | 3.7 |
| | 4 HR | 4.4 |
| *C. albicans* | 1 HR | NA |
| | 4 HR | NA |
| *F. solani* | 1 HR | NA |
| | 4 HR | NA |

**TABLE 24**

| Ingredients Example 24 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.100 |
| HPMC | | 0.400 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 4.6 |
| | 4 HR | >4.8 |
| *P. aeruginosa* | 1 HR | 4.7 |
| | 4 HR | >4.9 |
| *S. marcescens* | 1 HR | 3.4 |
| | 4 HR | 4.1 |
| *C. albicans* | 1 HR | NA |
| | 4 HR | NA |
| *F. solani* | 1 HR | NA |
| | 4 HR | NA |

**TABLE 25**

| Ingredients Example 25 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.020 |
| Pluronic^{®} F127 | | 2.500 |
| Pluronic^{®} P123 | | 0.250 |
| HPMC | | 0.400 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | >4.8 |
| | 4 HR | >4.8 |
| *P. aeruginosa* | 1 HR | >4.9 |
| | 4 HR | >4.9 |
| *S. marcescens* | 1 HR | 3.9 |
| | 4 HR | >4.1 |
| *C. albicans* | 1 HR | NA |
| | 4 HR | NA |
| *F. solani* | 1 HR | NA |
| | 4 HR | NA |

**TABLE 26**

| Ingredients Example 26 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.100 |
| HPMC | | 0.100 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | >4.3 |
| | 4 HR | >4.8 |
| *P. aeruginosa* | 1 HR | >4.7 |
| | 4 HR | >4.7 |
| *S. marcescens* | 1 HR | >4.9 |
| | 4 HR | >4.9 |
| *C. albicans* | 1 HR | 4.5 |
| | 4 HR | >4.7 |
| *F. solani* | 1 HR | 3.2 |
| | 4 HR | 3.8 |

**TABLE 27**

| Ingredients Example 27 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.250 |
| HPMC | | 0.100 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | >4.8 |
| | 4 HR | >4.8 |
| *P. aeruginosa* | 1 HR | >4.7 |
| | 4 HR | >4.7 |
| *S. marcescens* | 1 HR | >4.9 |
| | 4 HR | >4.9 |
| *C. albicans* | 1 HR | >4.7 |
| | 4 HR | >4.7 |
| *F. solani* | 1 HR | 3.8 |
| | 4 HR | >4.4 |

**TABLE 28**

| Ingredients Example 28 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.100 |
| HPMC | | - |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 3.7 |
| | 4 HR | >4.5 |
| *P. aeruginosa* | 1 HR | >4.7 |
| | 4 HR | >4.7 |
| *S. marcescens* | 1 HR | >4.9 |
| | 4 HR | >4.9 |
| *C. albicans* | 1 HR | 3.6 |
| | 4 HR | >4.7 |
| *F. solani* | 1 HR | 2.8 |
| | 4 HR | 3.8 |

**TABLE 29**

| Ingredients Example 29 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.250 |
| HPMC | | 0.100 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 4.4 |
| | 4 HR | >4.9 |
| *P. aeruginosa* | 1 HR | 3.9 |
| | 4 HR | >4.6 |
| *S. marcescens* | 1 HR | 1.9 |
| | 4 HR | >4.6 |
| *C. albicans* | 1 HR | 1.8 |
| | 4 HR | 3.1 |
| *F. solani* | 1 HR | 2.1 |
| | 4 HR | 3.4 |

**TABLE 30**

| Ingredients Example 30 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.100 |
| HPMC | | - |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 3.7 |
| | 4 HR | >4.9 |
| *P. aeruginosa* | 1 HR | 3.3 |
| | 4 HR | >4.6 |
| *S. marcescens* | 1 HR | 2 |
| | 4 HR | >4.6 |
| *C. albicans* | 1 HR | 2.9 |
| | 4 HR | >4.8 |
| *F. solani* | 1 HR | 1.4 |
| | 4 HR | 3.1 |

**TABLE 31**

| Ingredients Example 31 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.250 |
| HPMC | | 0.100 |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 3.4 |
| | 4 HR | >4.8 |
| *P. aeruginosa* | 1 HR | 2.6 |
| | 4 HR | 4.3 |
| *S. marcescens* | 1 HR | 3.0 |
| | 4 HR | >4.7 |
| *C. albicans* | 1 HR | 2.7 |
| | 4 HR | 3.2 |
| *F. solani* | 1 HR | 3.1 |
| | 4 HR | 4.6 |

**TABLE 32**

| Ingredients Example 32 | | %W/W |
|---|---|---|
| Boric Acid | | 0.850 |
| Sodium Phosphate (Monobasic) | | 0.150 |
| Sodium Phosphate (Dibasic) | | 0.310 |
| HAP (30%) | | 0.100 |
| Sodium Chloride | | 0.072 |
| Polymer JR | | 0.010 |
| Pluronic^{®} F127 | | 2.500 |
| Pluronic^{®} P123 | | 0.100 |
| HPMC | | - |
| PHMB HCl | | 4.0 ppm |
| Alexidine 2HCl | | 4.0 ppm |
| pH = 6.8- 7.2 | | |
| Osmolality (mOsmo/Kg) = 240-280 | | |
| *S. aureus* | 1 HR | 3.1 |
| | 4 HR | 4.4 |
| *P. aeruginosa* | *1 HR* | 2.5 |
| | 4 HR | 3.8 |
| *S. marcescens* | 1 HR | 2.7 |
| | 4 HR | 2.9 |
| *C. albicans* | 1 HR | 2.2 |
| | 4 HR | 3 |
| *F. solani* | 1 HR | 1.8 |
| | 4 HR | 3.1 |

### Example 33 - Making the Formulations

Table 33 lists the ingredients of the base formulation for the examples.

**Table 33: Base Formulation**

| Base Formulation | |
|---|---|
| Ingredient | %W/W |
| Sodium Chloride | 0.047 |
| Boric Acid | 0.85 |
| Sodium Phosphate (Monobasic) | 0.15 |
| Sodium Phosphate (Dibasic) | 0.31 |
| HAP | 0.1 |
| Polymer JR^{®} | 0.02 |
| Pluronic & Tetronic Copolymers | See Individual Formulations |
| Alexidine 2HCL | 3.0-5.0ppm |
| PH = 6.9- 7.1 | |
| Osmo.(mOsmo/Kg) = 220-300 | |

Table 34 shows actual surfactant concentrations of Formulations I to IV. All formulations are prepared by combining the respective amounts of the base formulation, surfactants with water. The formulations are filtered thereafter.

**Table 34: Compositions of Formulations I to IV**

| Formulation # Base Formulation Plus | | I | II | III | IV | V | VI | VII |
|---|---|---|---|---|---|---|---|---|
| Surfactants | HLB | | | | | | | |
| | | 3% | 3% | 4.5% | 4.5% | 3% | 2.5% | |
| Pluronic^{®} | 22 | | | | | | | |
| F127 | | | | | | | | |
| | | | 0.1% | | 0.1% | 0.05% | 0.25% | |
| Pluronic^{®} | 15 | | | | | | | |
| P105 | | | | | | | | |
| | | 1.5% | 1.5% | | | 1.5% | 1.25% | |
| Tetronic^{®} | 24 | | | | | | | |
| 1107 | | | | | | | | |

### Example 34 - Lipid cleaning and Toxicity Studies

Lipid cleaning studies were done based on a spectrophotometric measurement of the suspension, which includes the mixture of an orange dye (Sudan I) with cholesterol. Ten ml volume of formulations was tested for their ability of dissolving the lipid for 24 hour in room temperature. The higher the absorbance values, the higher the lipid cleaning efficacy of the formulations. Addition of 0.1% of Pluronic^{®} P105 (Formula IV: HLB value of 15) has a statistically increased the lipid cleaning values from the control composition (Formula III).

Toxicity data was generated using a cell culture model for predicting the ocular irritation potential of new contact lens care compositions (Na-Fluorescent permeability assay). All these formulations have shown permeability values below the level of control formulation I. The control formulation I is a currently marketed and safe ophthalmic care product.

**Table 35: Formulations I to IV Lipid Cleaning and Toxicity**

| Formulation # | I | II | III | IV |
|---|---|---|---|---|
| Lipid Cleaning Value | 330 | 390 | 594 | 707 |
| Toxicity (Fluorescent Unit) | 49 | 35 | 45 | 48 |

### Example 35 - Preservative Efficacy

Table 36 shows the results of the effect of preservative efficacy of formulations I to IV. Preservative efficacy was tested according to the procedures disclosed above. Each of formulations I to IV passed the preservative efficacy test.

**Table 36: Formulations I to IV Preservative Efficacy**

| Formulation # | | I | II | III | IV |
|---|---|---|---|---|---|
| PE Test Results | | | | | |
| *S. aureus* | | | | | |
| | 14 day | Passed | Passed | Passed | Passed |
| | 28 day | Passed | Passed | Passed | Passed |
| *P. aeruginosa* | | | | | |
| | 14 day | Passed | Passed | Passed | Passed |
| | 28 day | Passed | Passed | Passed | Passed |
| | | | | | |
| *E. coli* | | | | | |
| | 14 day | Passed | Passed | Passed | Passed |
| | 28 day | Passed | Passed | Passed | Passed |
| | | | | | |
| *C. albicans* | | | | | |
| | 14 day | Passed | Passed | Passed | Passed |
| | 28 day | Passed | Passed | Passed | Passed |
| | | | | | |
| *A. niger* | | | | | |
| | 14 day | Passed | Passed | Passed | Passed |
| | 28 day | Passed | Passed | Passed | Passed |
| | | | | | |

### Example 36 - Dose Response for P105 in Pluronic^{®} F127 and Tetronic^{®} 1107

Formulations I, II, V and VI were prepared. The lipid cleaning test was performed according to the procedure above for lipid cleaning. The higher the lipid cleaning value represents a higher surfactant activity. The results show that a significant increase in lipid cleaning of the control formulation (Formulation I) occurred when 0.05 wt.% to 0.1 wt.% of Pluronic^{®} P105 is added.

**Table 37:**

| **Formulation #** | | **I** | **II** | **V** | **VI** |
|---|---|---|---|---|---|
| Surfactants | HLB | | | | |
| | | 3% | 3% | 3% | 3% |
| Pluronic^{®} | 22 | | | | |
| F127 | | | | | |
| | | | 0.1% | 0.05% | 0.01% |
| Pluronic^{®} | 15 | | | | |
| P105 | | | | | |
| | | 1.5% | 1.5% | 1.5% | 1.5% |
| Tetronic^{®} | 24 | | | | |
| 1107 | | | | | |
| Lipid Cleaning Value | | 528 | 575 | 547 | 527 |

These data show a dose response and threshold limit for Pluronic^{®} P105 in a solution containing 3% Pluronic^{®} F127 and 1.5% Tetronic^{®} 1107.

**Table 38**

| Formulation # | | **II** | **VII** |
|---|---|---|---|
| Surfactants | HLB | | |
| | | 3% | 2.5% |
| Pluronic^{®} | 22 | | |
| F127 | | | |
| | | 0.1% | 0.25 % |
| Pluronic^{®} | 15 | | |
| P105 | | | |
| | | 1.5% | 1.25 % |
| Tetronic^{®} | 24 | | |
| 1107 | | | |
| Lipid Cleaning Value | | 581 | 591 |

These data show that you can have a lower total surfactant concentration and maintain lipid cleaning with the use of increased concentration of Pluronic^{®} P105.

As demonstrated above, the compositions disclosed herein that include a disinfecting amount of one or more biguanide antimicrobial agents and Pluronic^{®} P123 or Pluronic^{®} P105 are useful in contact lens care solutions for the rapid disinfection of RGP contact lenses. As may be appreciated, a disinfecting amount of antimicrobial agent is an amount that will at least partially reduce the microorganism population in the formulations employed. Preferably, a disinfecting amount is that which will reduce the microbial burden of representative bacteria by three log orders in four hours and one log order for representative fungi. Most preferably, a disinfecting amount is an amount which will eliminate the microbial burden of more than 10 cfu per tested microorganism on a contact lens, when used according to its regimen for the recommended soaking time as established by ISO (International Standards for Ophthalmic Optics)/FDA Stand-Alone Procedures for Disinfection Test (ISO/DIS 14729; 2001). Typically, such agents are present in concentrations ranging from about 0.00001 to about 0.5 percent weight/volume (w/v), and more preferably, from about 0.00003 to about 0.5 percent w/v. A preservative amount of antimicrobial agent is that amount that prevents biologic deterioration of substances or devices with which the compositions are used. Preservative amounts of antimicrobial agent in the subject compositions is about 0.0001 to about 5.0 weight percent, more preferably about 0.001 to about 1.0 weight percent and most preferably about 0.025 to about 0.50 weight percent.

As stated above, contact lenses are cleaned without the need for manual rubbing and rapidly disinfected by contacting the lens with a solution of the type disclosed herein. This is accomplished by simply soaking or immersing a contact lens in several milliliters of the subject solution. Preferably, the contact lens is permitted to soak in the solution for a period of at least one to four hours. The contact lens is then removed from the solution, optionally rinsed with the same or a different solution, such as a preserved isotonic saline solution, and then replaced on the eye.

Solutions containing one or more compositions of the present invention may be formulated into specific contact lens care products for use, as is customary in the field of ophthalmology. Such products include but are not limited to wetting solutions, soaking solutions, cleaning and conditioning solutions, as well as multipurpose lens care solutions and in-eye cleaning and conditioning solutions.

Solutions containing one or more compositions of the type disclosed herein may be formulated into specific products for disinfecting medical devices such as, for example, but not limited to, contact lenses.

Products containing one or more compositions of the type disclosed herein may be formulated for preservation against microbial contamination such as, for example, but not limited to, ophthalmic solutions, pharmaceuticals, artificial tears and comfort drops. Solutions containing one or more compositions of the type disclosed herein may be formulated into specific products for preserving medical devices from microbial contamination such as, for example, but not limited to, products formulated for the storage of contact lenses.

While the present invention has been described and illustrated by reference to particular embodiments, those of ordinary skill in the art will appreciate that the invention lends itself to variations not necessarily illustrated herein. For this reason, then, reference should be made solely to the appended claims for purposes of determining the true scope of the present invention.

### Examples 37-40 - Regimen Testing of Compositions with PHMB, Alexidine and Pluronic^{®} P123

A four-hour no rub and no rinse (NR/NR) regimen using 10 ml of four multipurpose solutions, with and without a 10 second shaking step (ss), is conducted on RGP lenses and tested against *Candida albicans* ATCC 10231, which is known to be the most resistant of all five microorganisms. (The RGP lenses are Boston ES^{®}, available from Bausch & Lomb of Rochester, NY, and are produced from enflufocon A.) The compositions of the four multipurpose solutions of Examples 37-40 are shown in Tables 39 and 40 below.

The performance requirement for the regimen calls for recovery of less than or equal to 10 colony-forming units (CFU) from each lens and filter combination for each test organism. The test results for the regimens are set forth below in Tables 39 and 40. All the compositions of the present invention meet the regimen criteria with *C. albicans*.

A Stand-Alone Biocidal study using 10% organic soil is also conducted on the four multipurpose solutions of Examples 37-40, as described in Tables 39 and 40, to show the overall antimicrobial efficacy of the four sample multipurpose solutions according to the present invention. The organic soil is a mixture of heat shocked *Saccharomyces cerevisiae* [1 X 10⁷] mixed, per ml, with Serum Bovine Albumin. To obtain a 10% concentration, the mixture is diluted 1 part to 9 parts with PBS. The solutions are tested against *Pseudomonas aeruginosa* ATCC 9027, *Staphylococcus aureus* ATCC 6538, *Serratia marcescens* ATCC13880, *Candida albicans* ATCC 10231 and *Fusarium solani* ATCC 36031.

The results of the Stand-Alone Biocidal study are also set forth below in Tables 39 and 40. The primary criteria for a given disinfection period, corresponding to a potential minimum recommended disinfection period for bacteria, is not less than 3.0 logs within the given disinfection period. The number of mold and yeast recovered per ml must be reduced by a mean value of not less than 1.0 log within the minimum recommended disinfection time with no increase at four times the minimum recommended disinfection time (24 hours). All four multipurpose solutions in Examples 37-40 meet the regimen criteria with all five microorganisms.

The data from Examples 37-40 demonstrate the effective antimicrobial profile for the NR/NR RGP multipurpose solutions of the present invention for both stand-alone biocidal testing and a no rub/no rinse regimen.

| | | TABLE 39 | |
|---|---|---|---|
| | | | |
| Ingredients (w/w%) | | Example 37 | Example 38 |
| Propyl-EDTA-OH | | 1.0 | 1.0 |
| Pluronic^{®} F38 | | 1 | 1 |
| Tetronic^{®} 908 | | 1 | 1 |
| Pluronic^{®} P123 | | 0.2 | 0.2 |
| PVP-30K | | 1 | 1 |
| EDTA (2Na+) | | 0.04 | 0.025 |
| Glycerin | | 1.1 | 1.1 |
| Polymer JR^{®} 30 M | | 0.0075 | 0.02 |
| Trehalose | | 0.2 | 0.2 |
| Alexidine | | 4.5 | 4.5 |
| PHMB (112ppm) | | | |
| HPMC | | 0.12 | 0.12 |
| pH | | 7.35 | 7.38 |
| Osmolarity (mOsmo/Kg) | | 222 | 209 |

| Regimen | | NR/NR | NR/NR |
|---|---|---|---|
| | | 4h soak/10ml/10ss (RGP) | 4h soak/10ml/10ss (RGP) |
| | | C. albicans (S. cerevisiae) | C. albicans (S. cerevisiae) |
| | | 0,0,0 | 0,0,0 |
| | | 4h soak/10ml/NS (RGP) | 4h soak/10ml/NS (RGP) |
| | | C. albicans (S. cerevisiae) | C. albicans (S. cerevisiae) |
| | | 0,0,0 | 0,0,0 |
| Inoculum Control (cfu/ml) | | C. albicans | C. albicans |
| | | 6.5x10⁷ | 5.9x10⁷ |
| Lens Control (cfu/lens) (n=2) | | 8.7x10⁵ | 6.7x10⁵ |
| | | 8.8x10⁵ | 7.1x10⁵ |
| Stand-Alone Biocidal 10%OS | | | |
| *S. aureus* | 1 hr | >4.7 | >4.7 |
| | 4 hr | >4.7 | >4.7 |
| *P. aeruginosa* | 1 hr | >4.7 | >4.6 |
| | 4 hr | >4.7 | >4.6 |
| *S. marcescens* | 1 hr | >4.6 | >4.8 |
| | 4 hr | >4.6 | >4.8 |
| *C. albicans* | 1 hr | 3.9 | >4.5 |
| | 4 hr | >4.7 | >4.5 |
| *F. solani* | 1 hr | >4.1 | >4.2 |
| | 4 hr | >4.1 | >4.2 |

| | | | |
|---|---|---|---|
| CFU = colony forming units 10ss=10 seconds shake Log Reduction, >= 100% kill NS=no shake NR/NR = no rub, no rinse | | | |

| | | TABLE 40 | |
|---|---|---|---|
| | | | |
| Ingredients (w/w%) | | Example 39 | Example 40 |
| Propyl-EDTA-OH | | 1.0 | 1.0 |
| Pluronic F38 | | 1 | 1 |
| Tetronic 908 | | 1 | 1 |
| Pluronic^{®} P123 | | 0.2 | 0.2 |
| PVP-30K | | 1 | 1 |
| EDTA (2Na+) | | 0.05 | 0.1 |
| Glycerin | | 1.1 | 1.1 |
| Polymer JR 30 M | | 0.02 | 0.02 |
| Trehalose | | 0.2 | 0.2 |
| Alexidine | | 4.5 | 4.5 |
| PHMB (112ppm) | | | |
| HPMC | | 0.12 | 0.12 |
| pH | | 7.35 | 7.31 |
| Osmolarity (mOsmo/Kg) | | 206 | 211 |
| Regimen | | NR/NR | NR/NR |
| | | 4h soak/10ml/10ss (RGP) | 4h soak/10ml/10ss (RGP) |
| | | C. albicans (S. cerevisiae) | C. albicans (S. cerevisiae) |
| | | 0,0,0 | 0,0,0 |
| | | 4h soak/10ml/NS (RGP) | 4h soak/10ml/NS (RGP) |
| | | C. albicans (S. cerevisiae) | C. albicans (S. cerevisiae) |
| | | 0,0,0 | 0,0,0 |
| Inoculum Control (cfu/ml) | | C. albicans | C. albicans |
| | | 5.9x10⁷ | 5.9x10⁷ |
| Lens Control (cfu/lens) (n=2) | | 6.7x10⁵ | 6.7x10⁵ |
| | | 7.1x10⁵ | 7.1x10⁵ |
| Stand-Alone Biocidal 10%OS | | | |
| *S. aureus* | 1 hr | >4.7 | >4.7 |
| | 4 hr | >4.7 | >4.7 |
| *P. aeruginosa* | 1 hr | >4.6 | >4.6 |
| | 4 hr | >4.6 | >4.6 |
| *S. marcescens* | 1 hr | >4.8 | >4.8 |
| | 4 hr | >4.8 | >4.8 |
| *C. albicans* | 1 hr | 4 | >4.5 |
| | 4 hr | >4.5 | >4.5 |
| *F. solani* | 1 hr | >4.2 | >4.2 |
| | 4 hr | >4.2 | >4.2 |

| | | | |
|---|---|---|---|
| CFU = colony forming units Log Reduction, >= 100% kill NR/NR = no rub, no rinse 10ss = 10 second shake NS = no shake | | | |

### Examples 41-44 - Testing of Compositions with Pluronic^{®} P105

### Example 41 - Making the Formulations

Table 41 lists the ingredients of the base formulation for the examples.

**Table 41: Base Formulation**

| Base Formulation | |
|---|---|
| Ingredient | %W/W |
| Sodium Chloride | 0.047 |
| Boric Acid | 0.85 |
| Sodium Phosphate (Monobasic) | 0.15 |
| Sodium Phosphate (Dibasic) | 0.31 |
| HAP | 0.1 |
| Polymer JR | 0.02 |
| Pluronic & Tetronic Copolymers | See Individual Formulations |
| Alexidine 2HCL | 3.0-5.0ppm |
| PH = 6.9- 7.1 | |
| Osmo.(mOsmo/Kg) = 220-300 | |

Table 42 shows actual surfactant concentrations of Formulations I to IV. All formulations are prepared by combining the respective amounts of the base formulation, surfactants with water. The formulations are filtered thereafter.

**Table 42: Compositions of Formulations I to IV**

| Formulation # Base Formulation Plus | | I | II | III | IV | V | VI | VII |
|---|---|---|---|---|---|---|---|---|
| Surfactants | HLB | | | | | | | |
| | | 3% | 3% | 4.5% | 4.5% | 3% | 2.5% | |
| Pluronic^{®} F127 | 22 | | | | | | | |
| | | | 0.1% | | 0.1% | 0.05% | 0.25% | |
| Pluronic^{®} P105 | 15 | | | | | | | |
| | | 1.5% | 1.5% | | | 1.5% | 1.25% | |
| Tetronic^{®} 1107 | 24 | | | | | | | |

### Example 42 - Lipid cleaning and Toxicity Studies

Lipid cleaning studies were done based on a spectrophotometric measurement of the suspension, which includes the mixture of an orange dye (Sudan I) with cholesterol. Ten ml volume of formulations was tested for their ability of dissolving the lipid for 24 hour in room temperature. The higher the absorbance values, the higher the lipid cleaning efficacy of the formulations. Addition of 0.1% of Pluronic^{®} P105 (Formula IV: HLB value of 15) has a statistically increased the lipid cleaning values from the control composition (Formula III).

Toxicity data was generated using a cell culture model for predicting the ocular irritation potential of new contact lens care compositions (Na-Fluorescent permeability assay). All these formulations have shown permeability values below the level of control formulation I. The control formulation I is a currently marketed and safe ophthalmic care product.

**Table 43: Formulations I to IV Lipid Cleaning and Toxicity**

| Formulation # | I | II | III | IV |
|---|---|---|---|---|
| Lipid Cleaning Value | 330 | 390 | 594 | 707 |
| Toxicity (Fluorescent Unit) | 49 | 35 | 45 | 48 |

### Example 43 - Preservative Efficacy

Table 44 shows the results of the effect of preservative efficacy of formulations I to IV. Preservative efficacy was tested according to the procedures disclosed above. Each of formulations I to IV passed the preservative efficacy test.

**Table 44: Formulations I to IV Preservative Efficacy**

| Formulation # | | I | II | III | IV |
|---|---|---|---|---|---|
| PE Test Results | | | | | |
| *S. aureus* | | | | | |
| | 14 day | Passed | Passed | Passed | Passed |
| | 28 day | Passed | Passed | Passed | Passed |
| *P. aeruginosa* | | | | | |
| | 14 day | Passed | Passed | Passed | Passed |
| | 28 day | Passed | Passed | Passed | Passed |
| | | | | | |
| *E. coli* | | | | | |
| | 14 day | Passed | Passed | Passed | Passed |
| | 28 day | Passed | Passed | Passed | Passed |
| | | | | | |
| *C. albicans* | | | | | |
| | 14 day | Passed | Passed | Passed | Passed |
| | 28 day | Passed | Passed | Passed | Passed |
| | | | | | |
| *A. niger* | | | | | |
| | 14 day | Passed | Passed | Passed | Passed |
| | 28 day | Passed | Passed | Passed | Passed |
| | | | | | |

### Example 44 - Dose Response for Pluronic^{®} P105 in Pluronic^{®} F127 and Tetronic^{®} 1107

Formulations I, II, V and VI were prepared. The lipid cleaning test was performed according to the procedure of Example II for lipid cleaning. The higher the lipid cleaning value represents a higher surfactant activity. The results show that a significant increase in lipid cleaning of the control formulation (Formulation I) occurred when 0.05 wt.% to 0.1 wt.% of Pluronic^{®} P105 is added.

**Table 45:**

| **Formulation #** | | **I** | **II** | **V** | **VI** |
|---|---|---|---|---|---|
| Surfactants | HLB | | | | |
| | | 3% | 3% | 3% | 3% |
| Pluronic^{®} | 22 | | | | |
| F127 | | | | | |
| | | | 0.1% | 0.05% | 0.01% |
| Pluronic^{®} | 15 | | | | |
| P105 | | | | | |
| | | 1.5% | 1.5% | 1.5% | 1.5% |
| Tetronic^{®} | 24 | | | | |
| 1107 | | | | | |
| Lipid Cleaning Value | | 528 | 575 | 547 | 527 |

These data show a dose response and threshold limit for Pluronic^{®} P105 in a solution containing 3% Pluronic^{®} F127 and 1.5% Tetronic^{®} 1107.

**Table 46**

| Formulation # | | **II** | **VII** |
|---|---|---|---|
| Surfactants | HLB | | |
| | | 3% | 2.5% |
| Pluronic^{®} | 22 | | |
| F127 | | | |
| | | 0.1% | 0.25 |
| Pluronic^{®} | 15 | | % |
| P105 | | | |
| | | 1.5% | 1.25 |
| Tetronic^{®} | 24 | | % |
| 1107 | | | |
| Lipid Cleaning Value | | 581 | 591 |

These data show that you can have a lower total surfactant concentration and maintain lipid cleaning with the use of increased concentration of Pluronic^{®} P105.

As demonstrated above, the compositions disclosed herein that include a disinfecting amount of one or more biguanide antimicrobial agents and Pluronic^{®} P123 or Pluronic^{®} P105 are useful in contact lens care solutions for the rapid disinfection of RGP contact lenses. As may be appreciated, a disinfecting amount of antimicrobial agent is an amount that will at least partially reduce the microorganism population in the formulations employed.

As stated above, contact lenses are cleaned without the need for manual rubbing and rapidly disinfected by contacting the lens with a solution of the type disclosed herein. This is accomplished by simply soaking or immersing a contact lens in several milliliters of the subject solution. Preferably, the contact lens is permitted to soak in the solution for a period of at least one to four hours. The contact lens is then removed from the solution, optionally rinsed with the same or a different solution, such as a preserved isotonic saline solution, and then replaced on the eye.

Solutions containing one or more compositions of the present invention may be formulated into specific contact lens care products for use, as is customary in the field of ophthalmology. Such products include but are not limited to wetting solutions, soaking solutions, cleaning and conditioning solutions, as well as multipurpose lens care solutions and in-eye cleaning and conditioning solutions.

Solutions containing one or more compositions of the type disclosed herein may be formulated into specific products for disinfecting medical devices such as, for example, but not limited to, contact lenses.

Products containing one or more compositions of the type disclosed herein may be formulated for preservation against microbial contamination such as, for example, but not limited to, ophthalmic solutions, pharmaceuticals, artificial tears and comfort drops. Solutions containing one or more compositions of the type disclosed herein may be formulated into specific products for preserving medical devices from microbial contamination such as, for example, but not limited to, products formulated for the storage of contact lenses.

While the present invention has been described and illustrated by reference to particular embodiments, those of ordinary skill in the art will appreciate that the invention lends itself to variations not necessarily illustrated herein. For this reason, then, reference should be made solely to the appended claims for purposes of determining the true scope of the present invention.

## Claims

1. A lens care composition comprising a disinfecting amount of at least one biguanide antimicrobial agent to disinfect a rigid gas permeable lens, at least two straight chain polyether surfactants based upon PEO-PPO-PEO, PPO-PEO-PPO, wherein one polyether surfactant has an HLB value of less than or equal to 15 and one polyether surfactant has an HLB value greater than or equal to 18.

2. The lens care composition of claim 1, wherein said straight chain, low HLB polyether surfactant has the chemical structure PEO₂₀PPO₇₀PEO₂₀.

3. The lens care composition of claims 1 and 2, wherein said straight chain, high HLB polyether surfactant has the chemical structure PEO₃₇PPO₅₆PEO₃₇.

4. The lens care composition of claims 1 to 3, wherein the biguanide antimicrobial agent is selected from the group consisting of PHMB HCl and Alexidine 2HCl and combinations thereof.

5. The lens care solution of claims 1 to 4, further comprising ethanolamine as a buffer.

6. The lens care solution of claims 1 to 5, further comprising glycerin as an osmotic agent.

7. The lens care composition of claims 1 to 6, wherein the composition is a no rub solution.

8. A method of cleaning and disinfecting a rigid gas permeable contact lens, the method comprising contacting the contact lens with a lens care solution for a period of time suitable to reduce or eliminate a microbial burden on said lens, the lens care solution comprising at least one biguanide antimicrobial agent to disinfect a rigid gas permeable lens, at least two straight chain polyether surfactants based upon PEO-PPO-PEO, PPO-PEO-PPO, wherein one polyether surfactant has an HLB_value of less than or equal to 15 and one polyether surfactant has an HLB value greater than or equal to 18.

9. The method of claim 8, wherein the method provides complete cleaning of the lens such that digital rubbing of the lens is not necessary to clean the lens.

10. The method of claims 8 and 9, wherein the straight chain, low HLB polyether surfactant has the chemical structure PEO₂₀PPO₇₀PEO₂₀.

11. The method of claims 8 to 10, wherein the straight chain, high HLB polyether surfactant has the chemical structure PEO₃₇PPO₅₆PEO₃₇.

12. The method of claims 8 to 11, wherein the biguanide is selected from the group consisting of PHMB HCl and Alexidine 2HCl.

13. The method of claim 12, wherein the biguanide is PHMB HCl.

14. The method of claims 8 to13, wherein the lens care solution further includes ethanolamine as a buffer.

15. The method of claims 8 to 14, wherein the lens care solution further includes glycerin as an osmotic agent.

16. The ophthalmic composition of claims 1 to 4 further comprising borate and phosphate as a buffer system.

17. The method of claims 8 to 13, wherein the lens care solution further comprises borate and phosphate as a buffer system.

18. The ophthalmic composition of claims 1 to 4 and 16 further comprising an oligosaccharide selected from hydroxypropylmethyl cellulose or hyaluronic acid.

19. The method of claims 8 to 13 and 17, wherein the lens care solution further comprises an oligosaccharide selected from hydroxypropylmethyl cellulose or hyaluronic acid.

## Patentansprüche

1. Eine Kontaktlinsenpflegezusammensetzung beinhaltend eine desinfizierende Menge von mindestens einem biguanidbasierten antimikrobiellen Wirkstoff zum Desinfizieren einer starren, gasdurchlässigen Linse, mindestens zwei geradkettige Polyethertenside basierend auf PEO-PPO-PEO, PPO-PEO-PPO, wobei ein Polyethertensid einen HLB-Wert von weniger als oder gleich 15 aufweist und ein Polyethertensid einen HLB-Wert größer als oder gleich 18 aufweist.

2. Die Kontaktlinsenpflegezusammensetzung gemäß Anspruch 1, wobei genanntes geradkettiges Polyethertensid mit niedrigem HLB die chemische Struktur PEO₂₀PPO₇₀PEO₂₀ besitzt.

3. Die Kontaktlinsenpflegezusammensetzung gemäß der Ansprüche 1 und 2, wobei genanntes geradkettiges Polyethertensid mit hohem HLB die chemische Struktur PEO₃₇PPO₅₆PEO₃₇ besitzt.

4. Die Kontaktlinsenpflegezusammensetzung gemäß der Ansprüche 1 bis 3, wobei der biguanidbasierte antimikrobielle Wirkstoff ausgewählt wird aus der Gruppe bestehend aus PHMB HCl und Alexidin 2HCl und Kombinationen hiervon.

5. Die Kontaktlinsenpflegezusammensetzung gemäß der Ansprüche 1 bis 4, ferner enthaltend Ethanolamin als Puffer.

6. Die Kontaktlinsenpflegezusammensetzung gemäß der Ansprüche 1 bis 5, ferner enthaltend Glycerin als ein osmotisches Agens.

7. Die Kontaktlinsenpflegezusammensetzung gemäß der Ansprüche 1 bis 6, wobei die Zusammensetzung eine "no rub"-Lösung ist.

8. Ein Verfahren zur Reinigung und Desinfektion einer starren, gasdurchlässigen Kontaktlinse, das Verfahren beinhaltend das in Kontakt bringen der Kontaktlinse mit einer Kontaktlinsenpflegezusammensetzung für einen Zeitraum geeignet zum Verringern oder Beseitigen einer mikrobiellen Belastung auf genannter Linse, die Kontaktlinsenpflegezusammensetzung beinhaltend mindestens einen biguanidbasierten antimikrobiellen Wirkstoff zum Desinfizieren einer starren, gasdurchlässigen Linse, mindestens zwei geradkettige Polyethertenside basierend auf PEO-PPO-PEO, PPO-PEO-PPO, wobei ein Polyethertensid einen HLB-Wert von weniger als oder gleich 15 aufweist und ein Polyethertensid einen HLB-Wert größer als oder gleich 18 aufweist.

9. Das Verfahren gemäß Anspruch 8, wobei das Verfahren eine vollständige Reinigung der Linse bietet, so dass das Reiben der Linse mit den Fingern nicht erforderlich zum Reinigen der Linse ist.

10. Das Verfahren gemäß der Ansprüche 8 und 9, wobei das geradkettige Polyethertensid mit niedrigem HLB die chemische Struktur PEO₂₀PPO₇₀PEO₂₀ besitzt.

11. Das Verfahren gemäß der Ansprüche 8 bis 10, wobei das geradkettige Polyethertensid mit hohem HLB die chemische Struktur PEO₃₇PPO₅₆PEO₃₇ besitzt.

12. Das Verfahren gemäß der Ansprüche 8 bis 11, wobei das Biguanid ausgewählt wird aus der Gruppe bestehend aus PHMB HCl und Alexidin 2HCl.

13. Das Verfahren gemäß Anspruch 12, wobei das Biguanid PHMB HCl ist.

14. Das Verfahren gemäß der Ansprüche 8 bis 13, wobei die Kontaktlinsenpflegezusammensetzung ferner Ethanolamin als Puffer enthält.

15. Das Verfahren gemäß der Ansprüche 8 bis 14, wobei die Kontaktlinsenpflegezusammensetzung ferner Glycerin als ein osmotisches Agens enthält.

16. Die ophthalmische Zusammensetzung gemäß der Ansprüche 1 bis 4, ferner enthaltend Borat und Phosphat als Puffersystem.

17. Das Verfahren gemäß der Ansprüche 8 bis 13, wobei die Kontaktlinsenpflegezusammensetzung ferner Borat und Phosphat als Puffersystem enthält.

18. Die ophthalmische Zusammensetzung gemäß der Ansprüche 1 bis 4 und 16, ferner enthaltend ein Oligosaccharid ausgewählt aus Hydroxypropylmethylcellulose oder Hyaluronsäure.

19. Das Verfahren gemäß der Ansprüche 8 bis 13 und 17, wobei die Kontaktlinsenpflegezusammensetzung ferner ein Oligosaccharid ausgewählt aus Hydroxypropylmethylcellulose oder Hyaluronsäure enthält.

## Revendications

1. Composition d'entretien pour lentilles de contact qui comprend une quantité désinfectante d'au moins un agent antimicrobien biguanide destiné à désinfecter une lentille de contact rigide perméable aux gaz, au moins deux surfactants polyéther à chaîne droite à base de PEO-PPO-PEO, PPO-PEO-PPO, un surfactant polyéther ayant une valeur de HLB inférieure ou égale à 15 et un surfactant polyéther ayant une valeur de HLB supérieure ou égale à 18.

2. Composition d'entretien pour lentilles de contact selon la revendication 1, dans laquelle ledit surfactant polyéther à chaîne droite et faible HLB a la structure chimique PEO₂₀PPO₇₀PEO₂₀.

3. Composition d'entretien pour lentilles de contact selon les revendications 1 et 2, dans laquelle ledit surfactant polyéther à chaîne droite et HLB élevée a la structure chimique PEO₃₇PPO₅₆PEO₃₇.

4. Composition d'entretien pour lentilles de contact selon les revendications 1 à 3, dans laquelle l'agent antimicrobien biguanide est choisi dans le groupe constitué par le PHMB HCl et l'alexidine 2HCl ainsi que des combinaisons de ceux-ci.

5. Solution d'entretien pour lentilles de contact selon les revendications 1 à 4, comprenant en outre de l'éthanolamine en tant que tampon.

6. Solution d'entretien pour lentilles de contact selon les revendications 1 à 5, comprenant en outre de la glycérine en tant qu'agent osmotique.

7. Composition d'entretien pour lentilles de contact selon les revendications 1 à 6, la composition étant une solution n'impliquant pas la nécessité de frotter.

8. Procédé de nettoyage et de désinfection d'une lentille de contact rigide et perméable aux gaz, le procédé comprenant la mise en contact de la lentille de contact avec une solution d'entretien pour lentilles de contact pendant une durée adaptée pour réduire ou éliminer une charge microbienne sur ladite lentille de contact, la solution d'entretien pour lentilles de contact comprenant au moins un agent antimicrobien biguanide pour désinfecter une lentille de contact rigide perméable au gaz, au moins deux surfactants polyéther à chaîne droite à base de PEO-PPO-PEO, PPO-PEO-PPO, un surfactant polyéther ayant une valeur de HLB inférieure ou égale à 15 et un surfactant polyéther ayant une valeur de HLB supérieure ou égale à 18.

9. Procédé selon la revendication 8, dans lequel le procédé permet le nettoyage complet de la lentille de contact de telle manière qu'il n'est pas nécessaire de frotter la lentille de contact avec les doigts pour la nettoyer.

10. Procédé selon les revendications 8 et 9, dans lequel le surfactant polyéther à chaîne droite et faible HLB a la structure chimique PEO₂₀PPO₇₀PEO₂₀.

11. Procédé selon les revendications 8 à 10, dans lequel le surfactant polyéther à chaîne droite et HLB élevée a la structure chimique PEO₃₇PPO₅₆PEO₃₇.

12. Procédé selon les revendications 8 à 11, dans lequel le biguanide est choisi dans le groupe constitué par le PHMB HCl et l'alexidine 2HCl.

13. Procédé selon la revendication 12, dans lequel le biguanide est le PHMB HCl.

14. Procédé selon les revendications 8 à 13, dans lequel la solution d'entretien pour lentilles de contact comprend en outre de l'éthanolamine en tant que tampon.

15. Procédé selon les revendications 8 à 14, dans lequel la solution d'entretien pour lentilles de contact comprend en outre de la glycérine en tant qu'agent osmotique.

16. Composition ophtalmique selon les revendications 1 à 4, comprenant en outre du borate et du phosphate en tant que système de tampon.

17. Procédé selon les revendications 8 à 13, dans lequel la solution d'entretien pour lentilles de contact comprend en outre du borate et du phosphate en tant que système de tampon.

18. Composition ophtalmique selon les revendications 1 à 4 et 16, comprenant en outre un oligosaccharide choisi parmi l'hydroxypropylméthylcellulose ou l'acide hyaluronique.

19. Procédé selon les revendications 8 à 13 et 17, dans lequel la solution d'entretien pour lentilles de contact comprend en outre un oligosaccharide choisi parmi l'hydroxypropylméthylcellulose ou l'acide hyaluronique.
